# EUROPÄISCHE PATENTSCHRIFT

(11) **EP 2 985 261 B1**
(45) Veröffentlichungstag und Bekanntmachung des Hinweises auf die Patenterteilung: **28.09.2016**
(21) Anmeldenummer: 14180928.5
(22) Anmeldetag: 14.08.2014
(51) Int. Cl.: C01B 31/18, C07B 41/06, C07C 45/50, C07C 47/02

(54) **SILP-katalysierte Hydroformylierung mit CO2**
SILP-catalysed hydroformylation with CO2
Hydroformylation catalysé SILP avec CO2

(43) Veröffentlichungstag der Anmeldung: 17.02.2016
(73) Patentinhaber: Evonik Degussa GmbH, 45128 Essen (DE)
(72) Erfinder: JULIS, Dr. Jennifer,, 40479 Düssedorf (DE); HAHN, Dr., Hanna,, 41799 Duisburg-Baerl, (DE); FRANKE, Prof., Robert,, 45772 Marl, (DE); JESS, Dr., Andreas,, 95447 Bayreuh, (DE); KORTH, Dr., Wolfgang,, 95447 Bayreuth, (DE); FRITSCHI, Susanne,, 97318 Kitzingen (DE)

(56) Entgegenhaltungen:
- WO-A1-2012/041846
- DE-A1-102010 030 209

## Beschreibung

Die Erfindung betrifft ein Verfahren zur Herstellung von Aldehyden aus Alkenen und Kohlendioxid, bei welchem zunächst Kohlendioxid mit Wasserstoff im Wege einer Reverse-Wassergas-Shift-Reaktion zu Kohlenmonoxid und Wasser umgesetzt wird, und sodann mindestens ein Alken mit dem erhaltenen Kohlenmonoxid und mit Wasserstoff im Wege einer Hydroformylierung zu mindestens einem korrespondierenden Aldehyd umgesetzt wird, und bei welchem sowohl die Reverse-Wassergas-Shift-Reaktion, als auch die Hydroformylierung in Gegenwart eines Katalysator-Systems erfolgt, welches als SILP-Katalysator-System eine katalytisch wirksame Zusammensetzung umfasst, die in einer einen festen Träger benetzenden, ionischen Flüssigkeit gelöst ist.

Die Hydroformylierung - auch Oxo-Reaktion genannt - ermöglicht es, Olefine (Alkene) mit Synthesegas (das ist ein Gemisch aus Kohlenmonoxid und Wasserstoff) in Aldehyde umzusetzen. Die erhaltenen Aldehyde weisen dann entsprechend ein Kohlenstoff-Atom mehr auf als die eingesetzten Olefine. Aus jedem Olefin entsteht dabei ein korrespondierender Aldehyd. Durch anschließende Hydrierung der Aldehyde entstehen Alkohole, die aufgrund ihrer Genese auch "Oxo-Alkohole" genannt werden. Die Hydrierung der Aldehyde zu den korrespondierenden Alkoholen geschieht oft unmittelbar als Anschlussreaktion zur Hydroformylierung, sofern genug Wasserstoff angeboten wird. In einer technisch ausgeführten Hydroformylierung werden somit meist auch Oxo-Alkohole hergestellt. Die Aldehyde entstehen aber immer zumindest als Zwischenprodukt.

Grundsätzlich sind alle Olefine der Hydroformylierung zugänglich, in der Praxis werden jedoch meist solche Olefine als Substrat in der Hydroformylierung eingesetzt, die zwei bis 20 Kohlenstoffatome aufweisen. Da Oxo-Alkohole vielfältig eingesetzt werden können - etwa für die Herstellung von Weichmachern für PVC, als Detergentien in Waschmitteln und als Riechstoffe - wird die Hydroformylierung großindustriell praktiziert.

Eine gute Übersicht über den allgemeinen Stand der Hydroformylierung von Olefinen findet sich in
B. CORNILS, W. A. HERRMANN, "Applied Homogeneous Catalysis with Organometallic Compounds", Vol. 1 & 2, VCH, Weinheim, New York, 1996
und in
R. Franke, D. Selent, A. Börner, "Applied Hydroformylation", Chem. Rev., 2012 (112), S.5675-5732, DOI:10.1021/cr3001803

Das für die Hydroformylierung benötigte Synthesegas wird heutzutage industriell aus fossilen Energieträgern gewonnen. Dies kann etwa durch Vergasung von Kohle (Kaneko, T., Derbyshire, F., Makino, E., Gray, D., Tamura, M. and Li, K: Coal Liquefaction. Ullmann's Encyclopedia of Industrial Chemistry. 2012) oder durch Verdüsung einer flüssigen Kohlenstoffquelle wie schweres Heizöl mit Sauerstoff und Wasserstoff (EP1327607A2) erfolgen.

Leider gehört die bewährte Herstellung von Synthesegas aus fossilen Kohlenstoffquellen zu den industriellen Prozessen mit den höchsten spezifischen CO₂-Emissionen. Im Interesse der Verringerung des "Carbon-Footprint" eines hydroformylierenden Chemieunternehmens ist des daher, Alternativen zur Bereitstellung von Synthesegas zu finden.

Vielversprechend erscheint dabei, CO₂ als Kohlenstoffquelle zu nutzen. Kohlendioxid ist in der Luftatmosphäre vorhanden und fällt auch in Verbrennungsprozessen oder chemischen Reaktionen an. Es ist daher besonders leicht und kostengünstig verfügbar. Zudem wird der Entzug von CO₂ aus der Luftatmosphäre von der öffentlichen Hand gefördert.

Hinzu kommt, dass Kohlenmonoxid unbestritten giftig ist und sein Einsatz als Synthesebaustein daher auf wachsende Akzeptanzprobleme bei der Bevölkerung stößt. Auch aus diesem Grund wird nach Wegen gesucht, Alkene mit CO₂ statt mit CO zu hydroformylieren. Gelöst werden muss dabei insbesondere das Problem, dass Kohlendioxid deutlich reaktionsträger ist als Kohlenmonoxid.

Die CO₂-Fixierung bzw. die Reaktion von CO₂ mit Alkenen zu sauerstoffhaltigen Kohlenwasserstoffen kann direkt oder indirekt erfolgen.

Beispiele für die direkte Fixierung von CO₂ an Alkenen ist die Nickel-katalysierte Carboxylierung von Alkenen und die Eisen-, Nickel- oder Palladium-katalysierte Hydrocarboxylierung von Alkenen mit CO₂. In beiden Fällen entstehen als Produkt die entsprechenden Carbonsäuren. Diese können nur mit speziellen Reduktionsmitteln wie DIBAL-H zu den korrespondierenden Aldehyden reduziert werden, da die Gefahr der Überreduktion zu Alkoholen entsteht. Darüber hinaus müssen bei der direkten CO₂-Fixierung an Alkenen stöchiometrische Mengen empfindlicher organometallischer Reagenzien (Grignard-Reagenz, Et₂Zn) oder starke Basen verwendet werden.

CO₂ kann auch auf indirektem Wege über eine zweischrittige Reaktion an Alkenen fixiert werden. Hierbei wird zunächst Kohlendioxid in situ zu Kohlenmonoxid reduziert und anschließend mit dem Alken und Wasserstoff zum Aldehyd hydroformyliert. Im Gegensatz zur direkten CO₂-Fixierung kann auf die Verwendung stöchiometrischer Mengen an empfindlichen organometallischen Verbindungen oder starke Basen verzichtet werden. Zudem wird der entsprechende Aldehyd als Hauptprodukt gebildet, welches leicht sowohl zur Carbonsäuren oxidiert als auch zum Alkohol reduziert werden kann.

Aus diesem Grunde erscheint die zweischritte, indirekte Hydroformylierung mit CO₂ für die industrielle Praxis als das Mittel der Wahl.

Dieser Weg wurde vor allem von TOMINAGA und SASAKI beforscht und beschrieben:
- K. Tominaga, Y. Sasaki, K. Hagihara, T. Watanabe, M. Saito, Chem. Lett. 1994, 1391-1394;
- K. Tominaga, Y. Sasaki, J. Mol. Catal. A: Chem. 2004, 220, 159-165;
- K. Tominaga et al: Chem. Lett. 2004, 33, 14-15;
- K. Tominaga, Catal. Today 2006, 115, 70-72;

In der Patentliteratur findet sich dazu EP2206694B1, welches den Einsatz eines Katalysators auf Basis von Ruthenium und Kobalt beschreibt. Die katalytisch aktiven Metalle sind in einer ionischen Flüssigkeit gelöst.

Der indirekte, zweischrittige Weg über eine Reverse-Wassergas-Shift-Reaktion wird auch in DE102010030209A1 (=US20130178657A1) beschritten. Von dieser Schrift gehen die Erfinder als nächstliegenden Stand der Technik aus.

Hier wird nämlich CO₂ zunächst mit photokatalytisch gewonnenen H₂ in CO und H₂O umgesetzt, und das dabei gewonnene CO mit Wasserstoff und n-Buten zu Valeraldehyd hydroformyliert. Als Katalysator wird dabei ein so genanntes SILP-System verwendet:
Unter einem SILP (=Supported Ion Liquid Phase) - Katalysator-System versteht man ein Katalysator-System, welches ein festes poröses Trägermaterial umfasst, dessen Oberfläche mit einer ionischen Flüssigkeit benetzt ist. In der ionischen Flüssigkeit gelöst ist wiederum eine katalytisch wirksame Zusammensetzung wie zum Beispiel ein Metall/Organophosphor-Komplex. Auf diese Weise wird die homogen gelöste katalytisch wirksame Zusammensetzung über die ionische Flüssigkeit an dem festen Träger fixiert. Man erhält einen immobilisierten Homogenkatalysator, der sich wie ein Heterogenkatalysator handhaben lässt und die Abtrennung des Katalysators aus dem Reaktionsgemisch signifikant erleichtert. Ein SILP-Katalysator-System kombiniert damit die Vorteile eines aktiven Homogenkatalysators mit denen eines einfach zu handhabenden Heterogenkatalysators.

Daneben existieren Katalysatorsysteme, bei denen katalytisch aktive Zusammensetzungen zwar homogen in einer ionischen Flüssigkeit gelöst sind, aber diese nicht auf einem festen Träger immobilisiert sind. Dies sind keine SILP-Systeme im Sinne der Erfindung, da sie sich mangels eines festen Trägers nicht wie ein Heterogenkatalysator handhaben lassen.

Für die Hydroformylierung von n-Buten geeignete, Rhodium-basierte SILP-Systeme werden in WO2012041846A1 und von Jakuttis et al. (Angew. Chem. 2011, 123, 4584-4588) beschreiben. Ein solches System ist aber nicht zwangsläufig auch dafür geeignet, eine Reverse-Wassergas-Shift-Reaktion zu katalysieren.

Demgegenüber schlägt die DE102010030209A1 vor, für die Hydroformylierung ein SILP-System enthaltend multinukleare Ruthenium-Komplexe, insbesondere Ru₃(CO)₁₂ einzusetzen. Es wird deutlich gemacht, dass für die Reverse-Wassergas-Shift-Reaktion und für die Hydroformylierung dasselbe Katalysatorsystem (also ein SILP-Katalysator auf Basis von Ruthenium) eingesetzt werden sollte. Allerdings sind Ru-Systeme in der Flüssigphasen-Hydroformylierung Rh-Systemen hinsichtlich ihrer Performance oft unterlegen. Bei ähnlichen Katalysatorkomplexen ergibt sich für Rhodium eine um den Faktor 10⁵ gesteigerte Aktivität gegenüber Ruthenium (vgl. Beller et al, Angew. Chemie Int. Ed. 2013, 52, 2949-2953).

In Hinblick auf diesen Stand der Technik liegt der vorliegenden Erfindung die Aufgabe zu Grunde, ein für die zweischrittige Hydroformylierung mit CO₂ optimiertes Katalysatorsystem in SILP-Technologie anzugeben.

Gelöst wird diese Aufgabe dadurch, dass zwei unterschiedliche SILP-Katalysator-Systeme eingesetzt werden, nämlich ein erstes auf Basis von Ruthenium, in dessen Gegenwart die Reverse-Wassergas-Shift-Reaktion durchgeführt wird und ein zweites auf Basis von Rhodium, in dessen Gegenwart die Hydroformylierung durchgeführt wird.

Gegenstand der Erfindung ist mithin ein Verfahren zur Herstellung von Aldehyden aus Alkenen und Kohlendioxid, bei welchem zunächst Kohlendioxid mit Wasserstoff im Wege einer Reverse-Wassergas-Shift-Reaktion zu Kohlenmonoxid und Wasser umgesetzt wird, und sodann mindestens ein Alken mit dem erhaltenen Kohlenmonoxid und mit Wasserstoff im Wege einer Hydroformylierung zu mindestens einem korrespondierenden Aldehyd umgesetzt wird, und bei welchem sowohl die Reverse-Wassergas-Shift-Reaktion, als auch die Hydroformylierung in Gegenwart eines Katalysator-Systems erfolgt, welches als SILP-Katalysator-System eine katalytisch wirksame Zusammensetzung umfasst, die in einer einen festen Träger benetzenden, ionischen Flüssigkeit gelöst ist, wobei zwei unterschiedliche SILP-Katalysator-Systeme eingesetzt werden, nämlich ein erstes SILP-Katalysator-System, in dessen Gegenwart die Reverse-Wassergas-Shift-Reaktion durchgeführt wird, und ein zweites SILP-Katalysator-System, in dessen Gegenwart die Hydroformylierung durchgeführt wird, wobei das erste SILP-Katalysator-System einen in ionischer Flüssigkeit gelösten Ruthenium-Komplex als katalytisch wirksame Zusammensetzung umfasst, und wobei das zweite SILP-Katalysator-System einen in ionischer Flüssigkeit gelösten Rhodium-Komplex als katalytisch wirksame Zusammensetzung umfasst.

Die Grundidee der Erfindung besteht somit darin, die Hydroformylierung von Olefinen mit CO₂ und H₂ in Gegenwart zweier Katalysatoren im Festbettreaktor durchzuführen, wobei einer der Katalysatoren ein homogener Rutheniumkomplex ist, der mittels ionischer Flüssigkeit auf einem Träger immobilisiert wurde und der die Reverse-Wassergasshift-Reaktion katalysiert. Der andere Katalysator ist ein homogener Rhodiumkomplex, der ebenfalls mittels ionischer Flüssigkeit auf einem Träger immobilisiert wurde und der die Hydroformylierung katalysiert.

Grundsätzlich ermöglicht das erfindungsgemäße Verfahren die Rückführung von CO₂ in die chemische Wertschöpfungskette, indem das bisher benötigte Kohlenmonoxid, welches aus Synthesegas gewonnen wird, durch CO₂ ersetzt wird. Im Falle von Valeraldehyd kann so pro Tonne Aldehyd mehr als eine halbe Tonne CO₂ zurückgeführt werden. Darüber hinaus kann auf die Zuführung des giftigen Kohlenmonoxids von außen verzichtet werden, da dieses in der Reverse-Wassergas-Shift-Reaktion in situ aus CO₂ und H₂ gebildet wird und direkt mit dem Olefin zum Aldehyd weiterreagiert. Zusätzlich wird durch die heterogenisierten Katalysatorsysteme (SILP-Technologie) eine vereinfachte Rückgewinnung des Katalysators, insbesondere der darin enthaltenen Übergangsmetalle, im Vergleich zu klassischen homogenen Systemen ermöglicht.

Gegenüber der aus DE102010030209A1 bekannten kombinierten Verwendung eines SILP-Katalysator-Systems auf Ru-Basis sowohl für die Reverse-Wassergas-Shift-Reaktion als auch für die Hydroformylierung wird eine bessere Performance erzielt: Sowohl der Umsatz, als auch die Selektivität zum gewünschten Aldehyd können mit dem kombinierten Katalysatorsystem im Vergleich zum SILP-Katalysator auf Ru-Basis gesteigert werden. Versuche belegen, dass im Falle von 1-Buten als Substrat mit dem kombinierten SILP-Katalysatorystem SILP-9 und SILP-10 die Selektivität zum gewünschten Aldehyd im Vergleich zum einfachen Ru-SILP-System SILP-3 von 13 % auf über 99 % erhöht wird. Der Umsatz wird ebenfalls um das 2,5-fache gesteigert.

Gegenüber der Verwendung des in EP2206694B1 gezeigten Ru/Co-basierten Katalysators erleichtert die hier eingesetzte SILP-Technologie deutlich die Katalysatorabtrennung, da erfindungsgemäß die ionische Flüssigkeit mit den darin gelösten katalytisch aktiven Metallen auf einem festen Träger immobilisiert ist. Dies ist bei EP2206694B1 nicht der Fall, sodass praktisch ein homogenes Katalysatorsystem aus dem Produktgemisch abgetrennt werden muss, was technisch aufwendig ist.

Stattdessen wird vorliegend das Verfahren praktisch an zwei heterogenen Katalysatoren durchgeführt, die jeweils im Festbett (also als Schüttung) angeordnet sind: Dazu wird ein Gasgemisch, was die für die jeweiligen Reaktionsschritte benötigen Edukte enthält, nacheinander durch die beiden Festbetten geführt. Im ersten Festbett findet die Reverse-Wassergas-Shift-Reaktion statt, im zweiten Festbett die Hydroformylierung. Die Katalysatoren verbleiben immobilisiert in den Schüttungen, sodass die Produkte mit dem Gasgemisch aus der Schüttung abgezogen werden. Das Gasgemisch ändert somit über den Gesamtprozess ständig seine Zusammensetzung.

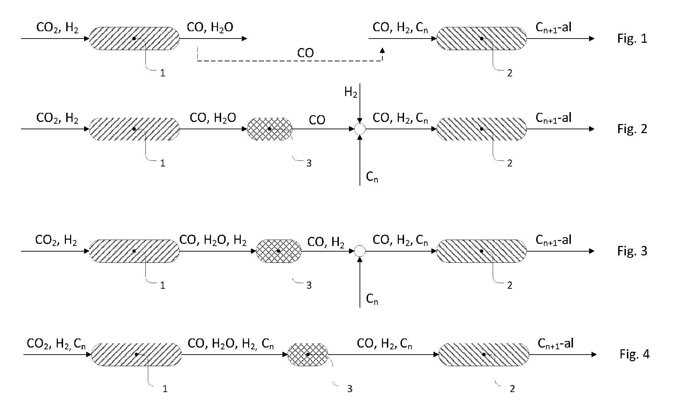

Es sind einige Ausführungsvarianten denkbar, wie die Edukte mit dem Gasgemisch in die Schüttungen eingebracht, und die Produkte mit dem Gasgemisch wieder abgezogen werden können. Diese werden später anhand der Figuren 1 bis 4 näher erläutert.

Wie bereits geschildert, weisen SILP-Systeme denknotwendig einen festen Träger auf, der mit einer ionischen Flüssigkeit benetzt ist. Denn dadurch wird die ionischen Flüssigkeit mit der darin gelösten katalytisch aktiven Substanz erst immobilisiert.

Es kann nicht ausgeschlossen werden, dass der feste Träger und die ionische Flüssigkeit ebenfalls katalytische Eigenschaften haben. Allerdings zeigen die Versuche dass für die katalytische Wirkung der in der ionischen Flüssigkeit gelöste Metall-Komplex deutlich relevanter ist. Deswegen wird dieser als die eigentliche katalytisch wirksame Zusammensetzung aufgefasst.

Der Träger besteht vorzugsweise aus einem porösen Material. Allerdings hat die Porosität nur einen geringen Einfluss auf das SILP-Katalysator-System, sodass hier auf genaue Angaben zur Porosität verzichtet wird. Geeignete Porenparameter wurden im Übrigen auch schon in WO2012041846A1 beschrieben.

Vorzugsweise werden als poröse Trägermaterialien solche eingesetzt, die inert bezüglich der übrigen Bestandteile des SILP-Katalysator-Systems und der in der Reaktion gegenwärtigen Stoffen, insbesondere den Edukten und Produkten, sind.

Bevorzugte Trägermaterialien sind anorganische, vorzugsweise oxidische Trägermaterialien. Als Trägermaterialien eigenen sich insbesondere Oxide von Aluminium, Silizium, Titan, Zirkon oder Aktivkohle oder Mischungen davon, die gegebenenfalls noch weitere Elemente aufweisen können. Gesichert geeignete Trägermaterialien sind Alumosilikate, Zeolithe, Al₂O₃ oder Siliziumdioxid. Besonders bevorzugt weist das Trägermaterial Siliziumdioxid auf oder besteht daraus. Konkret lässt sich pyrogene Kieselsäure oder Fällungskieselsäure als Träger gut verwenden.

Das erste SILP-Katalysator-System zur Umsetzung des CO₂ mit H₂ in CO und H₂O (Reverse-Wassergas-Shift-Reaktion) umfasst also:
- mindestens ein Trägermaterial, welches vorzugsweise porös ist;
- mindestens eine ionische Flüssigkeit;
- mindestens einen Ruthenium-Komplex
- optional noch zumindest einen Liganden.

Im Rahmen der vorliegenden Erfindung werden unter ionischen Flüssigkeiten- kurz IL genannt - Flüssigkeiten verstanden, die bei einem Druck von 101.325 Pa und einer Temperatur von kleiner 100 °C, vorzugsweise kleiner 50 °C und besonders bevorzugt kleiner/gleich 25 °C im flüssigen Aggregatzustand vorliegen und nahezu keinen messbaren Dampfdruck aufweisen, wie in Angew. Chem. Int. Ed. 2000, 39, 3772-3789 offenbart wird. Sie sind von inerten Lösungsmitteln, wie sie zur Herstellung des ersten und zweiten SILP-Katalysators benutzt werden, zu unterscheiden. Als IL können alle ionischen Flüssigkeiten verwendet werden, die die vorgenannten Eigenschaften aufweisen.

Bevorzugt werden als ionische Flüssigkeit solche verwendet, bei denen das Anion ausgewählt ist aus der Gruppe umfassend:
Tetrafluoroborat ([BF₄]⁻), Hexafluorophosphat ([PF₆]⁻), Dicyanamid ([N(CN)₂]⁻), Bis(trifluoromethylsulfonyl)imid ([NTf₂]⁻), Tricyanomethid ([C(CN)₃]⁻), Tetracyanoborat ([B(CN)₄]⁻), Halogenide (Cl⁻, Br⁻, F⁻, I⁻), Hexafluoroantimonat ([SbF₆]⁻), Hexafluoroarsenat ([AsF₆]⁻), Sulfat ([S0₄]²⁻), Tosylat ([C₇H₇SO₃]⁻), Triflat (CF₃SO₃⁻), Nonaflat ([C₄F₉SO₃-), Tris-(Pentafluoroethyl)-trifluorophosphat ([PF₃(C₂F₅)₃]-), Thiocyanat ([SCN]⁻), Carbonat ([CO₃]²⁻), [R^{A}-COO]⁻, [R^{A}-SO₃]⁻,[ R^{A}-SO₄]⁻ [R^{A}PO₄R^{B}]⁻ oder [(R^{A}-SO₂)₂N]⁻ mit R^{A} und R^{B} gleich oder ungleich, jeweils ein linearer oder verzweigter 1 bis 12 Kohlenstoffatome enthaltender aliphatischer oder alicyclischer Alkyl- oder Perfluoralkyl- oder ein C₅-C₁₈- substituierter Aryl-, C₅-C₁₈- substituierter Aryl-C₁-C₆-alkyl- oder C₁-C₆-Alkyl-C₅-C₁₈- substituierter ArylRest ist, der durch Halogenatome substituiert ein kann;
und das Kation ausgewählt ist aus der Gruppe umfassend:
   quaternäre Ammonium-Kationen der allgemeinen Formel [NR₁R₂R₃R₄]⁺ mit R₁, R₂, R₃, R₄ ausgewählt aus C₁-C₈-Alkylgruppen;
   Phosphonium-Kationen der allgemeinen Formel [PR₁R₂R₃R₄]⁺ mit R₁, R₂, R₃, R₄ ausgewählt aus C₁-C₆-Alkylgruppen;
   Imidazolium-Kationen der allgemeinen Formel (A), 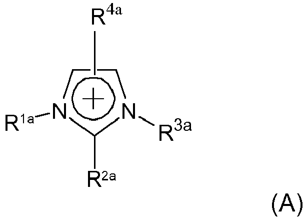
   wobei der Imidazol-Kern substituiert sein kann mit wenigstens einer Gruppe R^{na} mit n =1, 2, 3 oder 4, die ausgewählt ist aus C₁-C₈-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen;
   Pyridinium-Kationen der allgemeinen Formel (B), 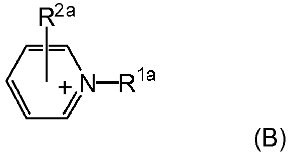
   wobei der Pyridin-Kern substituiert sein kann mit wenigstens einer Gruppe R^{na} mit n = 1 oder 2, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen;
   Pyrazolium-Kationen der allgemeinen Formel (C), 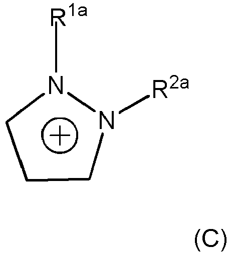
   wobei der Pyrazol-Kern substituiert sein kann mit wenigstens einer Gruppe R^{na} mit n = 1 oder 2, die ausgewählt ist aus C₁-C₆-Alkyl-, C₁-C₆-Alkoxy-, C₁-C₆- substituierte Aminoalkyl-,
   C₅-C₁₂- substituierte Aryl- oder C₅-C₁₂- substituierte Aryl-C₁-C₆-Alkylgruppen;
   Triazolium-Kationen der allgemeinen Formel (D), 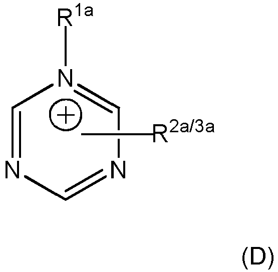 wobei der Triazol-Kern substituiert sein kann mit wenigstens einer Gruppe R^{na} mit n =1, 2, oder 3, die ausgewählt ist aus C₁-C₆-Alkyl-, C₁-C₆-Alkoxy-, C₁-C₆- substituierte Aminoalkyl-, C₅-C₁₂- substituierte Aryl- oder C₅-C₁₂- substituierte Aryl-C₁-C₆-Alkylgruppen.

Bevorzugte ionische Flüssigkeiten sind solche, die als Kation eine Imidazolium-Struktur der oben genannten Formel (A) aufweisen, wobei der Imidazol-Kern mit wenigstens einer Gruppe R^{na} mit n =1, 2, 3 oder 4 substituiert ist, ausgewählt unter C₁ - C₈-Alkylgruppen. Ganz besonders bevorzugt im ersten SILP-Katalysator-System ist die ionische Flüssigkeit ausgewählt aus der Gruppe umfassend:
1-Ethyl-3-methylimidazolium bis(triflouoromethansulfonyl)imide ([EMIM][Ntf2]),
1-Butyl-3-methylimidazolium bis(triflouoromethansulfonyl)imide ([BMIM][Ntf2]),
1-Ethyl-3-methylimidazolium chlorid ([EMIM]Cl),
1-Butyl-3-methylimidazolium-chlorid ([BMIM]Cl),
oder aus Mischungen daraus.

Es kann die reine halogenidhaltige IL verwendet werden oder Mischungsverhältnisse der halogenidhaltiger IL und nicht-halogenidhaltiger IL zwischen 1:0,001 bis 1:1000, bevorzugt 1:1. Die halogenid-haltige ionische Flüssigkeit ist für Reverse-Wassergas-Shift-Reaktion notwendig. Die nicht-halogenidhaltige ionische Flüssigkeit ermöglicht das Lösen der katalytisch aktiven Zusammensetzung. Das Mischungsverhältnis 1:1 ist das bevorzugte Mischungsverhältnis. Der Anteil an halogenidhaltiger IL kann aber auch deutlich geringer sein z.B. 1:9.

Der Ruthenium-Komplex Trirutheniumdodecacarbonyl (Ru3(CO)12) ist nachweislich zur Katalyse der Reverse-Wassergas-Shift-Reaktion geeignet und ist deswegen besonders bevorzugt.

Vorzugsweise weist die katalytisch wirksame Zusammensetzung des ersten SILP-Katalysatorsystems neben dem Ruthenium-Komplex noch mindestens einen Liganden auf. Der Ligand besteht aus Kohlenmonoxid, kann aber zusätzlich noch organische Verbindungen enthalten, wie z.B. 1,1-Bis(diphenylphosphin)methan und 2,2'-Bipyridin.

Das zweite SILP-Katalysator-System zur Umsetzung des Alkens mit CO₂ und H₂ in den korrespondierende Aldehyd CO (Hydroformylierung) umfasst:
- mindestens ein Trägermaterial, welches vorzugsweise porös ist;
- mindestens eine ionische Flüssigkeit;
- Rhodium;
- mindestens eine phosphorhaltige organische Verbindung als Ligand zur Bildung des Rhodium-Komplexes;
- optional ein oder mehrere organische Amine.

Als Träger innerhalb des zweiten SILP-Katalysator-Systems eignen sich dieselben Materialen, die oben zu dem ersten SILP-Katalysator-System erörtert wurden.

Zu der ionischen Flüssigkeit gilt dasselbe. Besonders bevorzugt ist im zweiten SILP-Katalysator-System ist die ionische Flüssigkeit 1-Ethyl-3-methylimidazolium bis(triflouoromethansulfonyl)imide ([EMIM][Ntf2]).

Besonders bevorzugt enthält die katalytisch wirksame Zusammensetzung des zweiten SILP-Katalystorsystems mindestens einen der folgenden Organophosphor-Liganden:
- [6,6'-((3,3'-di-tert-butyl-5,5'-dimethoxy-[1,1'-biphenyl]-2,2'-diyl)bis(oxy))bis(dibenzo[d,f][1,3,2]dioxaphosphepin)] gemäß Formel VII; 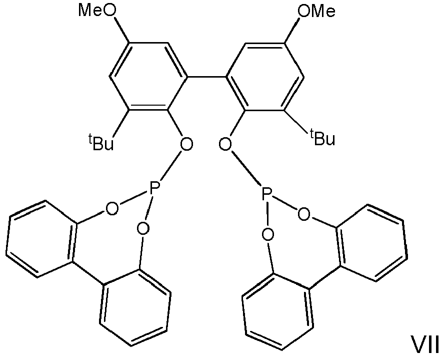
- [2,2'-((3,3'-di-tert-butyl-5,5'-dimethoxy-[1,1'-biphenyl]-2,2'-diyl)bis(oxy))bis(4,4,5,5-tetraphenyl-1,3,2-dioxaphospholane)] gemäß Formel VIII; 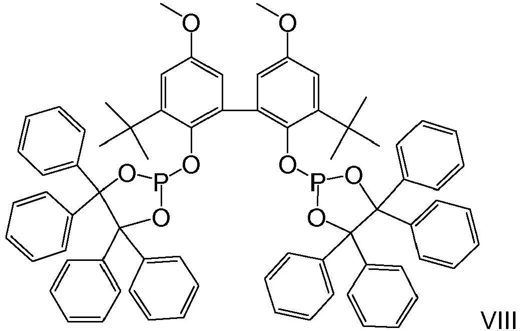
- Tris-(2,4-di-tert-butylphenyl)phosphit gemäß Formel IX; 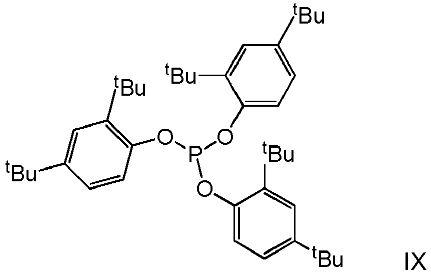
- Triphenylphosphin (PPh3);
- tri-sulfoniertes Triphenylphosphin (TPPTS).

Aufgrund ihrer Wassertoleranz werden besonders bevorzugt Phospine als Liganden in der Hydroformylierung eingesetzt. Deswegen umfasst das zweite SILP-Katalystor-System als Ligand besonders bevorzugt Triphenylphosphin (PPh3) oder tri-sulfoniertes Triphenylphosphin. Zwar sind diese Liganden weniger aktiv bzw. selektiv als die oben genannten Phosphite, allerdings sind diese in Gegenwart von Wasser hydrolyseanfällig, weswegen auf eine peinliche Entwässerung vor der Hydroformylierung geachtet werden muss. Die Phosphine hingegen hydrolysieren nicht so stark, sodass an die Entfernung von Reaktionswasser aus der ersten Stufe keine allzu hohen Anforderungen gestellt werden müssen.

Das Rh-Phosphin-System sollte vorzugsweise stabilisiert werden mit einem Stabilisator wie beispielsweise einem organischen Amin. Bevorzugt umfasst das verwendete organische Amin mindestend einen Rest mit einer 2,2,6,6-Tetramethylpiperidineinheit nach Formel III:

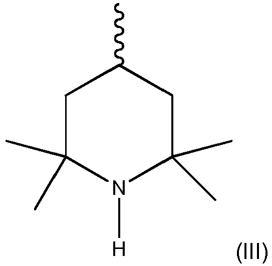

Besonders bevorzugt ist das organische Amin in der erfindungsgemäßen Zusammensetzung ausgewählt aus den Verbindungen der Formeln (IIIa) - (IIIh):

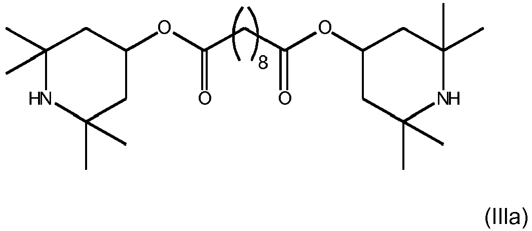

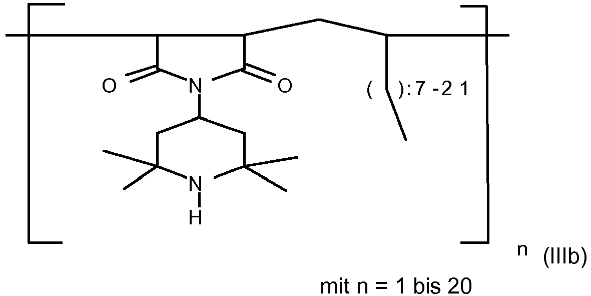

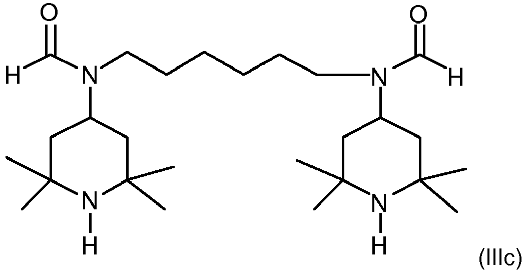

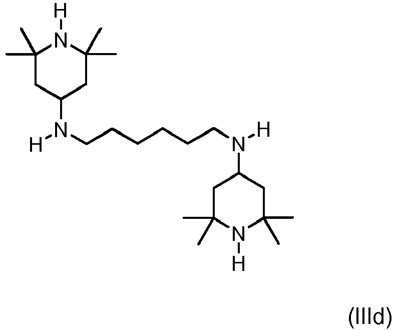

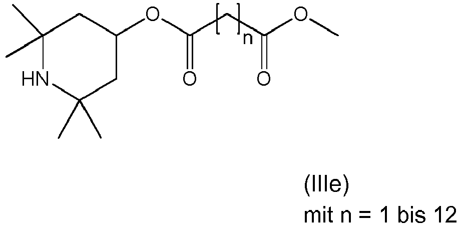

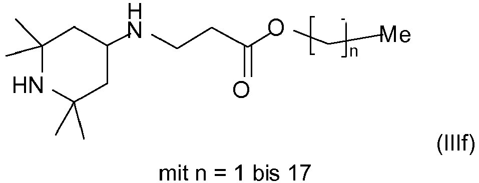

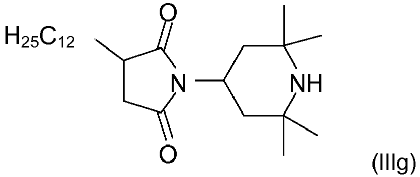

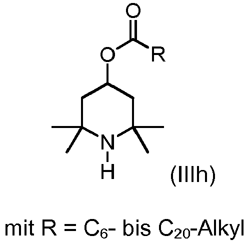

Grundsätzlich lassen sich alle hydroformylierbaren Alkene als Substrate für das vorliegende Verfahren einsetzen. Vorzugsweise werden Alkene eingesetzt, die zwei bis acht Kohlenstoffatome aufweisen. Ganz besonders bevorzugt werden Alkene mit zwei bis vier Kohlenstoffatomen eingesetzt, da diese sich noch in der Gasphase an dem SILP-Systemen hydroformylieren lassen. Versuche belegen den erfindungsgemäßen Einsatz von n-Buten (auch 1-Buten genannt), weswegen dieses Substrat besonders bevorzugt ist. Die Erkenntnisse aus dem Einsatz von n-Buten lassen sich aber problemlos auf andere Substrate übertragen.

Sofern Alkene mit vier Kohlenstoffatome (kurz C₄ wie n-Buten,) eingesetzt werden, so werden diese im Wege der Hydroformylierung zu den korrespondierenden Aldehyden mit fünf Kohlenstoffatomen (kurz: C₅-Aldehyde oder C₅-al) umgesetzt. Zu diesen gehören: n-Pentanal (Valeraldehyd), iso-Pentanal (Isovaleradelhyd), sec-Pentanal (2-Methylbutanal) und tert-Pentanal (Pivalaldehyd).

Der Gesamtprozess sieht dann wie folgt aus:
Erster Schritt (Reverse-Wassergas-Shift-Reaktion):

   CO₂ + H₂ -> CO + H₂O
Zweiter Schritt (Hydroformylierung):

   C₄ + CO + H₂ -> C₅-al
Gesamtreaktion:

   C₄+ CO₂ + 2 H₂ -> C₅-al + H₂O

Die so aus Kohlendioxid, Wasserstoff und n-Buten hergestellten Pentanale (C₅-al) eignen sich unter anderem als Ausgangsstoffe zur Erzeugung von Pentanolen, Pentansäuren und Pentylaminen. Durch Aldolkondensation und Totalhydrierung des Aldolkondensats können aus ihnen Decanole gewonnen werden, die Zwischenprodukte für die Herstellung von Weichmachern, Detergenzien und Schmiermitteln sind. Ein entsprechender Prozess ist in DE102009001594A1 beschreiben.

Die Hydroformylierung mit CO₂ am kombinierten Katalysatorsystem erfolgt bei 90 °C bis 150 °C, bevorzugt bei einer Temperatur von 100 °C bis 140 °C. Der Reaktionsdruck beträgt zwischen 10*10⁵ Pa bis 100*10⁵ Pa, bevorzugt bei 20*10⁵ Pa bis 50*10⁵ Pa, wobei das Verhältnis der Partialdrücke der einzelnen Komponenten Alken, CO₂ und H₂ 1:2:4 betragen kann.

Die Erfindung soll nun näher erläutert werden. Hierfür zeigen:
- Figur 1:: Verfahrensfließbild erste Ausführungsform (Basisverfahren);
- Figur 2:: Verfahrensfließbild zweite und dritte Ausführungsform;
- Figur 3:: Verfahrensfließbild vierte Ausführungsform;
- Figur 4:: Verfahrensfließbild fünfte Ausführungsform;
- Figur 5:: Verfahrensfließbild des Gasphasenprozesses zur Hydroformylierung von 1-Buten. Gasversorgung mit CO bzw. CO₂, H₂ und He; DMV1 bis DMV3: Druckminderungsventile; FI: Flow Indication (Durchflussanzeige); H1 bis H3: Heizung; MFC1 bis MFC3: Mass-Flow-Controller (Gasdurchflussregelung); PE: Probenentnahme, RM: Rotameter; RV1 bis RV4: Rückschlagventil; TI/TIC: Temperature Indication Controll (Temperaturanzeige und -regelung); V1 bis V9: Absperrventile und Dreiwegehahn; ÜSV: Überströmventil;
- Figur 6:: Festbettreaktor für die Hydroformylierung von 1 -Buten an einem SILP-Katalysator mit CO₂ und das Temperaturprofil über der Schüttung. FBR: Festbettreaktor, h: Höhe (Rkt: Reaktor, KS: Katalysatorschüttung), KS: Katalysatorschüttung, SN: Stahlnetz, TFR: Thermoelementführungsrohr

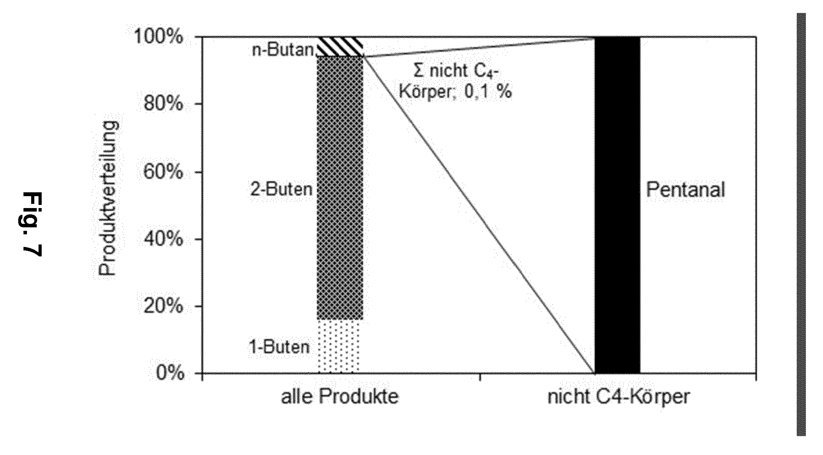

- Figur 7:: Produktverteilung bei einem Experiment unter Verwendung eines SILP Katalysators zur Umsetzung von CO₂ zu CO und eines Zweiten zur Hydroformylierung von 1-Buten im kontinuierlichen Gasphasenprozess; Reaktionsbedingungen: T = 120 °C, p = 21 bar, Vges = 6,9 L(norm)/h, p(Buten/CO₂/H₂) (2,6/4,1/7,3) bar; RWGS-SILP: Träger: Silica 60, IL: [EMIM]Cl, Katalysator: Ru₃(CO)₁₂, mSILP = 42,5, xRu(SILP) = 0,06 Gew.-%, α = 0,17, TRu = 10 min; Hydrof.-SILP: Träger: Silica 90, IL: [EMIM][NTf2], Katalysator: Rh(CO)₂(acac), PPh₃:Stabilisator:Ru = 37:97:1 [molar], mSILP = 2,8 g, xRh(SILP) = 0,3 Gew.-%, α = 0,1, TRh = 0,4 min;
- Figur 8:: Typische Produktverteilung der Hydroformylierung von 1-Buten an einem Ru-SILP-Katalysator im kontinuierlichen Gasphasenprozess mit CO₂ (andere: C1- bis C8-Alkane, ohne Butan); Reaktionsbedingungen: T = 120 °C, p = 21 bar, Vges = 8,1 L(norm)/h, p(Buten/CO₂/H₂) = (3,1/3,2/6,4) bar, T = 6,4 min; SILP: Träger: Silica 60, IL: [EMIM]Cl, Katalysator: Ru₃(CO)₁₂, mSILP = 32 g, xRu(SILP) = 0,06 Gew. %, α = 0,17;
- Figur 9:: Typische Produktverteilung der Hydroformylierung von 1-Buten an einem Ru-SILP-Katalysator mit Ligand im kontinuierlichen Gasphasenprozess mit CO₂; Reaktionsbedingungen: T = 125 °C, p = 21 bar, SILP: Träger: Silica 60, IL: [EMIM]Cl/[EMIM][Ntf2] 1:1, Katalysator: Ru₃(CO)₁₂; PPh₃ (SILP-2): Vges = 25,1 IN/h, p(Buten/CO₂/H₂) = (3/3/6,6) bar, T = 3,3 min; P(P^{t}Bu₂)₃ (SILP-5): Vges = 27,8 L(norm)/h, p(Buten/CO₂/H₂) = (3,1/3,2/6,4) bar, T = 3,0 min.

### Verfahrensvarianten

Eine allgemeine, erste Ausführungsvariante des Verfahrens zeigt die Figur 1 und umfasst die folgenden Schritte:
a) Beaufschlagen einer ersten Schüttung (1) enthaltend das erste SILP-Katalysator-System mit einem ersten Eduktgasstrom enthaltend zumindest Kohlendioxid (CO₂) und Wasserstoff (H₂);
b) Abziehen eines ersten Produktgasstroms enthaltend zumindest Kohlenmonoxid (CO) und Wasser (H₂O) aus der ersten Schüttung (1);
c) Beaufschlagen einer zweiten Schüttung (2) enthaltend das zweite SILP-Katalysator-System mit einem zweiten Eduktgasstrom enthaltend zumindest Kohlenmonoxid (CO), Wasserstoff (H₂) und Alken (Cₙ), wobei zumindest ein Teil des enthaltenden Kohlenmonoxids (CO) aus dem ersten Produktgasstrom stammt;
d) Abziehen eines zweiten Produktgasstroms enthaltend zumindest ein korrespondierendes Aldehyd (Cₙ₊₁-al) aus der zweiten Schüttung (2).

Das Verfahren läuft somit vollständig in der Gasphase ab, eine Katalysatorabtrennung erübrigt sich, da die beiden quasi festen SILP-Katalysatoren in ihren jeweiligen Schüttungen verbleiben und das Produktgemisch gasförmig die Schüttung verlässt. Die Durchführung ist damit verfahrenstechnisch sehr einfach und mithin auch industriell praktikabel.

Wesentlich ist, dass zumindest ein Teil des in der Hydroformylierung benötigten Kohlenmonoxids aus der ersten Reaktion stammt. Dies muss entsprechend überführt werden (gestrichelte Linie in Figur 1). Allerdings sollte das aus der ersten Schüttung abgezogene Produktgemisch enthaltend das CO nicht ohne weiteres als Feed für die zweite Stufe verwendet, werden, da es Wasser enthält.

Problematisch ist das in der Reverse-Wassergas-Shift-Reaktion entstehende Reaktionswasser, das flüssig oder gasförmig im ersten Produktgasgemisch enthalten sein kann. Da es in der Hydroformylierung stört, indem es unerwünschte Nebenprodukte bildet, die katalytisch aktive Substanz durch Hydrolyse des enthaltenden Liganden zerstört oder schlicht Reaktorvolumen verbraucht, ist es vor der Hydroformylierung zu entfernen.

Eine zweite, bevorzugte Ausführungsvariante sieht deswegen den in Figur 2 skizzierten Verfahrensablauf vor:
a) Beaufschlagen einer ersten Schüttung (1) enthaltend das erste SILP-Katalysator-System mit einem ersten Eduktgasstrom enthaltend zumindest Kohlendioxid (CO₂) und Wasserstoff (H₂);
b) Abziehen eines ersten Produktgasstroms enthaltend zumindest Kohlenmonoxid (CO) und Wasser (H₂O) aus der ersten Schüttung (1);
c) Entfernen zumindest eines Teils des im ersten Produktgasstrom enthaltenden Wassers (H₂O) mit Hilfe eines Adsorbers (3);
d) Beaufschlagen einer zweiten Schüttung (2) enthaltend das zweite SILP-Katalysator-System mit einem zweiten Eduktgasstrom enthaltend zumindest Kohlenmonoxid (CO), Wasserstoff (H₂) und Alken (Cₙ), wobei zumindest ein Teil des enthaltenden Kohlenmonoxids (CO) aus dem ersten Produktgasstrom stammt;
e) Abziehen eines zweiten Produktgasstroms enthaltend zumindest ein korrespondierendes Aldehyd (Cₙ₊₁-al) aus der zweiten Schüttung (2).

Die Entwässerung in Schritt c) mit Hilfe des Adsorbers (3) ist deswegen vorteilhaft, da der Adsorber auch wie die Katalysatoren im Festbett vorliegen kann, welcher von dem ersten Produktgasstrom durchströmt wird. Die adsorbtive Entwässerung ist damit verfahrenstechnisch einfach zu integrieren.

Es können reversible und/oder irreversible Adsorbentien verwendet werden. Bevorzugt wird in zunächst ein reversibler Adsorber durchströmt und danach ein irreversibler Adsorber als zweite Restentwässerungsinstanz. Die reversiblen Adsorber sind in parallelen Gefäßen angeordnet, die alternierend durchströmt und reaktiviert werden. Als Adsorbens eignet sich Silica.

Eine dritte und besonders bevorzugte Ausführungsvariante der Erfindung ist ebenfalls in Figur 2 dargestellt und umfasst die folgenden Schritte:
a) Beaufschlagen einer ersten Schüttung (1) enthaltend das erste SILP-Katalysator-System mit einem ersten Eduktgasstrom enthaltend zumindest Kohlendioxid (CO) und Wasserstoff (H₂);
b) Abziehen eines ersten Produktgasstroms enthaltend zumindest Kohlenmonoxid (CO) und Wasser (H₂O) aus der ersten Schüttung (1);
c) Entfernen zumindest eines Teils des im ersten Produktgasstrom enthaltenden Wassers (H₂O) mit Hilfe eines Adsorbers (3);
d) Zugabe von zumindest Alken (Cₙ) und Wasserstoff (H₂) in den entwässerten ersten Produktgasstrom unter Erhalt eines zweiten Eduktgasstroms;
e) Beaufschlagen einer zweiten Schüttung (2) enthaltend das zweite SILP-Katalysator-System mit dem zweiten Eduktgasstrom enthaltend zumindest Kohlenmonoxid (CO), Wasserstoff (H₂) und Alken (Cₙ), wobei zumindest ein Teil des enthaltenden Kohlenmonoxids (Cₒ) aus dem ersten Produktgasstrom stammt;
f) Abziehen eines zweiten Produktgasstroms enthaltend zumindest ein korrespondierendes Aldehyd (Cₙ₊₁) aus der zweiten Schüttung (2).

Der Vorteil dieses Verfahrensablaufes besteht darin, dass das Alken und der Wasserstoff, der in der Hydroformylierung benötigt wird, erst unmittelbar vor der zweiten Schüttung in den entwässerten CO-haltigen Produktgasstrom aus der ersten Schüttung zugegeben wird. Dies hat den Vorteil, dass die erste Schüttung nicht mit unnötigen Volumen belastet wird, wodurch die Apparate- und Katalysatorkosten geringer werden. Zudem wird die unerwünschte Nebenproduktbildung in der ersten Schüttung weitestgehend vermieden, da dort nur die zwingend erforderlichen Reaktionsteilnehmer anwesend sind.

Sollte das in der ersten Stufe gebildete CO nicht ausreichen, kann bei dieser Ausführungsform auch klassisch produziertes Synthesegas dem entwässerten ersten Produktstrom beigemengt werden. Dies ist in Figur 2 nicht dargestellt, kann aber gemeinsam mit dem H₂ am Vermischungspunkt (O) vor der zweiten Schüttung (2) erfolgen.

Da Wasserstoff als einziges Edukt in beiden Reaktionen benötigt wird, empfiehlt sich auch die in Figur 3 gezeigte, vierte Ausführungsform der Erfindung, bei der Wasserstoff im Überschuss der ersten Stufe zugeführt wird:
a) Beaufschlagen einer ersten Schüttung enthaltend das erste SILP-Katalysator-System (1) mit einem ersten Eduktgasstrom enthaltend zumindest Kohlendioxid (CO₂) und ein Überschuss an Wasserstoff (H₂);
b) Abziehen eines ersten Produktgasstroms enthaltend zumindest unumgesetzten Wasserstoff (H₂), Kohlenmonoxid (CO) und Wasser (H₂O) aus der ersten Schüttung (1);
c) Entfernen zumindest eines Teils des im ersten Produktgasstrom enthaltenden Wassers (H₂O) mit Hilfe eines Adsorbers (3);
d) Zugabe von zumindest Alken (Cₙ) in den entwässerten ersten Produktgasstrom unter Erhalt eines zweiten Eduktgasstroms;
e) Beaufschlagen einer zweiten Schüttung (2) enthaltend das zweite SILP-Katalysator-System mit dem zweiten Eduktgasstrom enthaltend zumindest Kohlenmonoxid (CO), Wasserstoff (H₂) und Alken (Cₙ), wobei zumindest ein Teil des enthaltenden Kohlenmonoxids (CO) aus dem ersten Produktgasstrom stammt;
f) Abziehen eines zweiten Produktgasstroms enthaltend zumindest ein korrespondierendes Aldehyd (Cₙ₊₁ -al) aus der zweiten Schüttung (2).

Überschuss an Wasserstoff bedeutet in diesem Zusammenhang, dass mehr Wasserstoff der ersten Stufe zugeführt wird, als von der Reverse-Wassergas-Shift-Reaktion benötigt wird. Der in der ersten Stufe unverbrauchte Wasserstoff wird dann in der zweiten Stufe umgesetzt. Der Überschuss in der Reverse-Wassergas-Shift-Reaktion sollte deswegen möglichst so kalkuliert sein, dass genügend Wasserstoff für die Hydroformylierung übrig bleibt.

Das erst in der zweiten Reaktionsstufe benötigte Alken wird unmittelbar vor dem Eintritt in die zweite Schüttung eingespeist, sodass Nebenreaktionen des Alkens in der ersten Schüttung vermieden werden. Vorteil dieses Verfahren ist die vereinfachte Einspeisung vor der zweiten Stufe, da hier nur Alken zugeführt wird.

Eine Zwischeneinspeisung lässt sich gänzlich vermeiden, wenn alle benötigten Edukte von Anfang an im Gasstrom enthalten sind. Ein solcher Verfahrensablauf ist in Figur 4 dargestellt:
a) Beaufschlagen einer ersten Schüttung (1) enthaltend das erste SILP-Katalysator-System mit einem ersten Eduktgasstrom enthaltend zumindest Kohlendioxid (CO₂), Alken (Cₙ) und ein Überschuss an Wasserstoff (H₂);
b) Abziehen eines ersten Produktgasstroms enthaltend zumindest unumgesetzten Wasserstoff (H2), Alken (Cₙ), Kohlenmonoxid (CO) und Wasser (H₂O) aus der ersten Schüttung (1);
c) Entfernen zumindest eines Teils des im ersten Produktgasstrom enthaltenden Wassers (H₂O) mit Hilfe eines Adsorbers (3) unter Erhalt eines zweiten Eduktgasstroms;
d) Beaufschlagen einer zweiten Schüttung (2) enthaltend das zweite SILP-Katalysator-System mit dem zweiten Eduktgasstrom enthaltend zumindest Kohlenmonoxid (CO), Wasserstoff (H₂) und Alken (Cₙ), wobei zumindest ein Teil des enthaltenden Kohlenmonoxids (CO) aus dem ersten Produktgasstrom stammt;
e) Abziehen eines zweiten Produktgasstroms enthaltend zumindest ein korrespondierendes Aldehyd (Cₙ₊₁) aus der zweiten Schüttung (2).

Nachteil dieser fünften Ausführungsvariante ist allerdings die Nebenproduktbildung und das große Gasvolumen in der ersten Stufe. Aus diesem Grunde ist dieser Verfahrensablauf für den industriellen Einsatz eher nachteilig.

Für Laboranwendungen kann dem Prozess auch ein Inertgas wie Helium zugemischt werden, welches die Dosierung der Edukte erleichtert.

### Aufbau des kontinuierlichen Gasphasenprozesses:

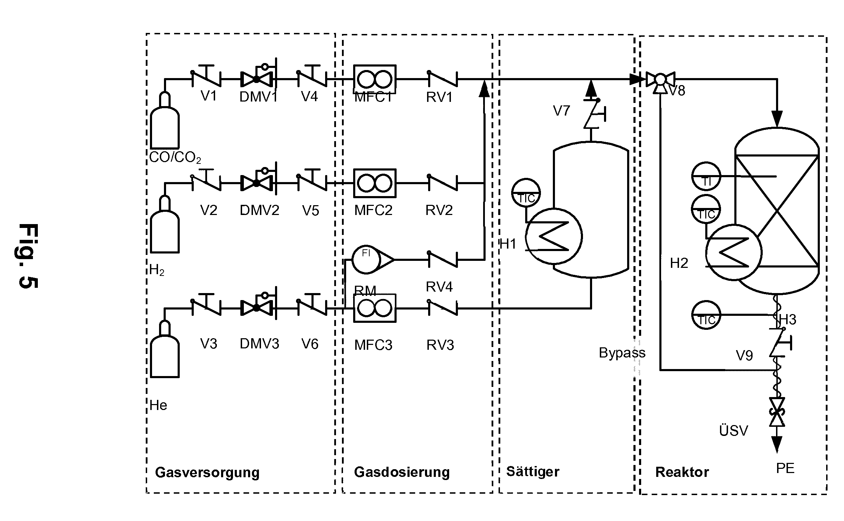

Die Versuchsanlage für den kontinuierlichen Gasphasenprozess zur Hydroformylierung von 1-Buten mit CO₂ lässt sich in vier Bereiche gliedern. Wie das Fließbild der Anlage in Figur 5 zeigt, handelt es sich dabei um die Gasversorgung, die Gasdosierung, den Sättiger und den Reaktor.

Die Gasversorgung besteht aus den Gasflaschen der benötigten Einsatzgase Wasserstoff (H₂), Helium (He) und Kohlendioxid (CO) bzw. Kohlenmonoxid (CO₂). Das ebenfalls benötigte 1-Buten gelangt nicht über die Gasversorgung, sondern über den Sättiger in den Reaktor. Ergänzt wird die Gasversorgung durch Absperrhähne (V1 - V3) direkt an den Gasflaschen, Druckminderungsventile (DMV1 - DMV3) und Absperrhähne (V4 - V6) an der Anlage. Diese Bauteile dienen der Sicherheit, um unkontrolliertes Ausströmen von Gas mit zu hohem Druck zu unterbinden. Die Gasdosierung erfolgt durch Gasdurchflussregler (MFC1 - MFC3), die durch Rückschlagventile (RV1 - RV3) gesichert sind. Der Heliumstrom kann zudem über eine zweite Leitung am Sättiger vorbei in die Anlage eingeleitet werden, was für eine Verdünnung der Reaktionsgase notwendig ist. Dieser Teilstrom wird über ein Rotameter (RM) geregelt und ein Rückschlagventil (RV4) gesichert.

Der Sättiger besteht aus einem beheizbaren (H1) Stahlgefäß, an dessen Kopf der mit 1-Buten abgesättigte Heliumstrom mit den anderen Reaktionsgasen zusammengeführt wird (V7). Der Sättiger ist wiederum mit einer mit 1-Buten gefüllten Gasflasche verbunden, die in Figur 5 jedoch nicht dargestellt ist.

Anschließend durchströmt das Gasgemisch den Reaktor (Festbettreaktor, FBR), ein Absperrventil (V9) und ein Überströmventil (ÜSV). Der Reaktor wird über die Heizung H2 auf die gewünschte Reaktionstemperatur geregelt (TIC). Auch der Reaktorausgang ist mit einem Heizregler (H3, TIC) ausgestattet, um eine Kondensation der Produkte zu vermeiden. Am Kopf des Reaktors befindet sich der Dreiwegehahn V8, über den der Gasstrom durch einen Bypass am Reaktor vorbei geleitet werden kann. Am Ausgang der Anlage können Proben aus dem Gasstrom für die Analytik entnommen werden (PE).

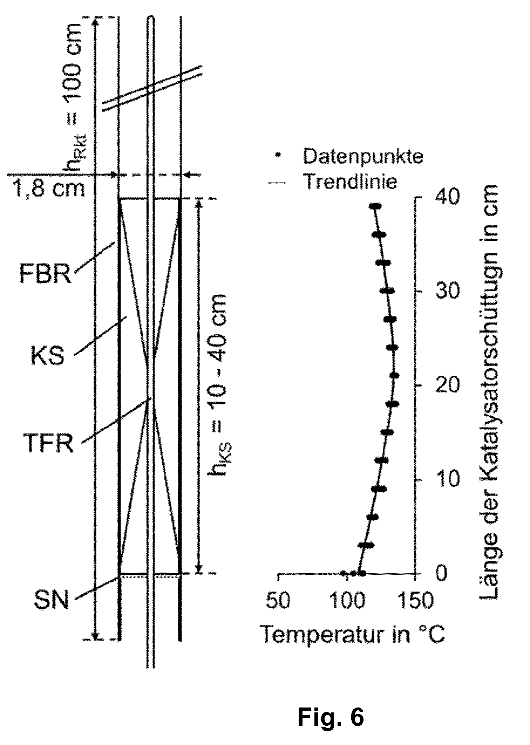

Der Reaktor selbst ist ein Rohrreaktor aus Stahl mit einem Innendurchmesser von 1,8 cm und einer Höhe von 100 cm. Er ist im Inneren mit einem engmaschigen Stahlnetz (SN) ausgestattet, auf dem die Katalysatorschüttung (KS) aufliegt. Die Reaktionstemperatur kann über ein Thermoelementführungsrohr (TFR) axial über den gesamten Reaktor einschließlich der Katalysatorschüttung erfasst werden. Der detaillierte Reaktoraufbau und das Temperaturprofil über der Katalysatorschüttung sind in Figur 6 dargestellt.

### Versuchsdurchführung

Vor der Inbetriebnahme werden die Regeleinheiten (MFC1 - MFC3, RM) kalibriert, um die gewünschten Volumenströme einzustellen.

Zu Beginn einer Versuchsreihe wird der Katalysator unter Inertatmosphäre in den Reaktor eingefüllt. Dafür wird der Reaktor am Kopf aufgeschraubt, am Rotameter (RM) ein Heliumstrom eingestellt und dieser über den Bypass von unten (V9 auf, ÜSV zu) durch den Reaktor geleitet. Zunächst wird der Katalysator SILP-10 für die Hydroformylierung von oben aus einem Argon durchströmten Schlenkkolben in den Reaktor eingeführt. Danach wird etwas Watte in den Reaktor gegeben, um den Katalysator räumlich zu trennen. Auf die Watteschichte wird Silica 90 (1.6 g) gegeben, um bei der Reverse-Wassergas-Shift-Reaktion entstandenes Wasser abzufangen. Die Silicaschicht wird ebenfalls mit Watte bedeckt. Abschließend wird der Katalysator SILP-9 für die Reverse-Wassergas-Shift-Reaktion eingefüllt. Anschließend kann der Reaktor wieder in der Anlage verschraubt und der Heliumstrom nun von oben über diesen geleitet werden (V9 auf, ÜSV auf). Unter dem Heliumstrom wird der Reaktor (H2), der Sättiger (H1) und der Reaktorausgang (H3) auf die gewünschte Temperatur aufgeheizt und durch langsames Schließen des ÜSV der Reaktionsdruck aufgebaut. Sind die Temperaturen erreicht, werden die Gasströme über den Bypass geleitet und über die Durchflussregelung (MFC1 - MFC3) die gewünschten Volumenströme eingestellt. Bei Bedarf kann eine Verdünnung des Einsatzgases über den zweiten Heliumstrom (RM) vorgenommen werden. Eine regelmäßige Überprüfung der Volumenströme erfolgt über die Messung mit einem Seifenblasenzählrohr. Nun kann eine Probe des Gasgemisches entnommen werden (PE), um die Eingangszusammensetzung zu ermitteln. Das Experiment selbst startet mit dem Umschalten der Gase vom Bypass auf den Reaktor. Während des gesamten Experimentes werden in regelmäßigen Abständen Proben in einem Gassammelrohr aufgefangen und mit einem Gaschromatographen analysiert.

Am Ende eines Experimentes wird die Förderung der Reaktionsgase eingestellt und die Heizungen abgestellt. Der Reaktor wird unter einem Heliumstrom abgekühlt.

Im Fallle von SILP-2, SILP-3 und SILP-5 als Katalysatorsystem wird analog verfahren. Da nur ein einziges Katalysatorsystem verwendet wird, wird auf die Watte- und Silicatrennschicht verzichtet und der Reaktor nach Befüllung mit dem Katalysator direkt wieder in der Anlage verschraubt.

### Herstellung und Charakterisierung der SILP-Katalysatoren

Die Herstellung der SILP-Katalysatoren SILP-2, SILP-3, SILP-5 und SILP-9 erfolgt gemäß Jakuttis et al. (Jakuttis, M., Schönweiz, A., Werner, S., Franke, R., Wiese, K.-D., Haumann, M., Wasserscheid, P., Angew. Chem. Int. Ed. 19 (2011), 4492-4495).

Die verwendeten Chemikalien (Katalysatoren, Liganden, ionische Flüssigkeiten, Trägermaterial (Silica) und Lösungsmittel (DCM)) werden, soweit notwendig, getrocknet und unter Inertgasatmosphäre gelagert. Soweit nicht anders angegeben, werden alle Arbeitsschritte unter Inertgasatmosphäre (Argon) durchgeführt.

Zunächst werden die notwendigen Mengen Katalysator in einen Schlenkkolben eingewogen und in DCM gelöst. Beim Katalysatorsystem SILP-2 wird zusätzlich der Ligand Triphenylphosphin (PPh₃) hinzugegegeben, beim Katalysatorsystem SILP-5 der Ligand Tri(2,4-di-*tert*-butylphenyl)phosphit (P(P^{t}Bu₂)₃). Die Lösung wird 5 min gerührt, die errechnete Menge IL zugegeben und das Gemisch erneut 5 min gerührt. Nach Zugabe der gewünschten Menge Silicagel wird das Lösungsmittel langsam unter Rühren im leichten Vakuum entfernt. Der fertige SILP-Katalysator wird bis zu seiner Verwendung unter inerten Bedingungen gelagert.

Als Katalysator wurde in den hier gezeigten Ergebnissen Trirutheniumdodecacarbonyl (Ru₃(CO)₁₂) verwendet. Die verwendeten ionischen Flüssigkeiten waren [EMIM][Ntf2], [BMIM][Ntf2], [EMIM]Cl und [BMIM]Cl sowie Mischungen daraus. Als inertes Trägermaterial diente Silica 60.

Für die Hydroformylierung von Buten mit dem aus der RWGS gewonnenem CO wurde SILP 10 nach Jakuttis et al. (Jakuttis, M., Schönweiz, A., Werner, S., Franke, R., Wiese, K.-D., Haumann, M., Wasserscheid, P., Angew. Chem. Int. Ed. 19 (2011), 4492-4495) hergestellt

Als katalytischer Metallkomplex dient hier (Acetylacetonato)dicarbonylrhodium (Rh(CO)₂(acac))) und als Ligand das PPh₃. Zusätzlich wird ein Stabilisator Bis(2,2,6,6 tetramethyl 4 piperidyl) sebacate (BTPS, C10H16O4 (C9H18N)2)) verwendet und als Träger kommt Silica 90 zum Einsatz. Die hergestellten sowie untersuchten SILP-Katalysatoren sowie ihre charakteristischen Merkmale sind in Tabelle 1 dargestellt.

**Tabelle 1**

| | m_{SILP} [g] | Katalysator | W_{Ru} [Gew.-%] | Ligand (L) | mol. Verhältnis L:Ru | IL | α | mol. Verhältnis IL:Cl⁻: Ru |
|---|---|---|---|---|---|---|---|---|
| SILP-2 | 54 | Ru₃(CO)₁₂ | 0,04 | A | 2,9:1 | [BMIM][Ntf₂]/Cl | 0,21 | 150:75:1 |
| SILP-3 | 32 | Ru₃(CO)₁₂ | 0,06 | -- | -- | [EMIM]Cl | 0,17 | 123:123:1 |
| SILP-5 | 55 | Ru₃(CO)₁₂ | 0,04 | B | 3:1 | [BMIM][Ntf₂]/Cl | 0,2 | 151:76:1 |
| SILP-9 | 43 | Ru₃(CO)₁₂ | 0,06 | -- | -- | [EMIM]Cl | 0,17 | 122:122:1 |
| SILP-10 | 3 | Rh(CO)₂(acac) | (W_{Rh}) 0,3 | A + BTPS | 37:97(BTPS):1(Rh) | [EMIM][Ntf₂] | 0,1 | 16 : 0 : 1 (Rh) |

| | | | | | | | | |
|---|---|---|---|---|---|---|---|---|
| A = Triphenylphosphin B =Tris(2,4-ditert-butylphenyl)phsophit Tabelle 1 | | | | | | | | |

Die Charakterisierung der SILP-Katalysatoren erfolgt über die Bestimmung der Oberfläche und Porenradienverteilung der Partikel mittels BET-Analytik nach DIN 66132 Einpunktmethode. Es wird eine abgewogene Menge des Materials in einem Probenröhrchen 4 h bei Raumtemperatur evakuiert. Anschließend wird das Röhrchen in das Gerät Gemin 2360 der Firma micromeritics eingebaut und die Messung gestartet.

### Ergebnis:

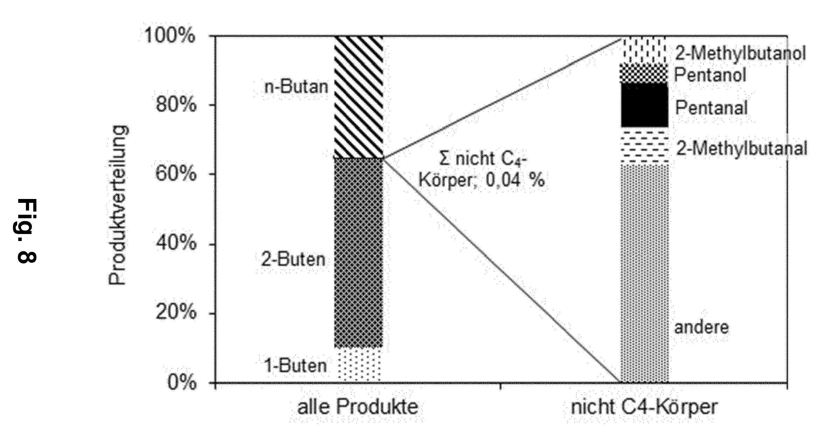

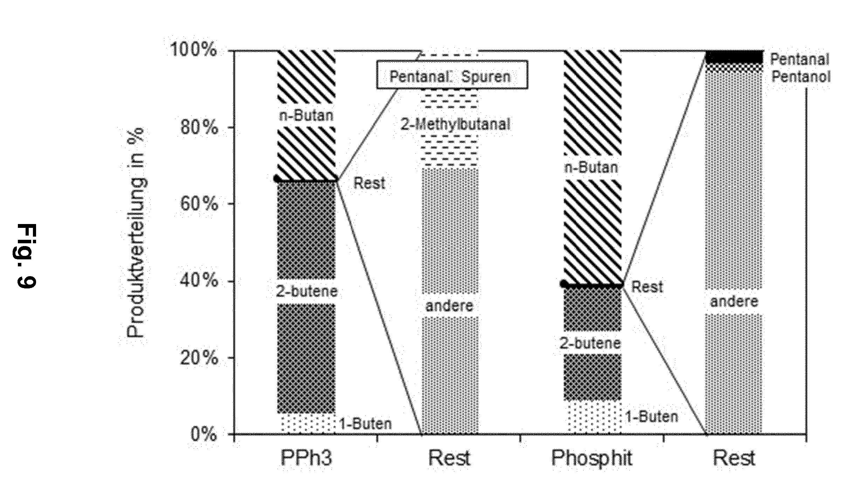

Die Ergebnisse der Hydroformylierung von 1-Buten mit CO₂ an einem kombinierten Katalysatorkonzept aus Rhodium und Ruthenium sind in Abbildung 3 wiedergegeben. Die Ergebnisse der Hydrofromylierung von 1-Buten mit CO₂ an einem Ruthenium Katalysator sind in den Figuren 8 und 9 wiedergegeben. Die Produktverteilung zeigt Tabelle 2.

**Tabelle 2: Produktverteilung**

| Komponente | SILP-2 | | SILP-3 | | SILP-5 | | SILP-9 und SILP-10 | |
|---|---|---|---|---|---|---|---|---|
| 1-Buten [%] | 5,3 | | 35,0 | | 8,5 | | 16,3 | |
| 2-Buten [%] | 60,9 | | 54,9 | | 30,3 | | 78,2 | |
| n-Butan [%] | 33,8 | | 10,1 | | 61,1 | | 5,4 | |
| 2-Methylbutanol [%] | 0,01 | - | 0,04 | 7,9 | 0,09 | - | 0,1 | - |
| Pentanol [%] | | - | | 5,3 | | 2,3 | | - |
| Pentanal [%] | | Spuren | | 13,2 | | 3,5 | | 100 |
| 2-Methylbutanal [%] | | 30,8 | | 10,5 | | - | | - |
| Andere [%] | | 69,2 | | 63,2 | | 94,2 | | - |

| | | | | | | | | |
|---|---|---|---|---|---|---|---|---|
| Andere: Octan, Hexan, Pentan, Penten, Propan, Ethan, Methan | | | | | | | | |

Die Ergebnisse in Tabelle 2 zeigen, dass die Hydroformylierung von 1-Buten mit CO₂ an einem kombinierten Katalysatorsystem aus Rhodium und Ruthenium mit einer Selektivität über 99 % zum gewünschten Aldehyd 1-Pentanal möglich ist. Im Falle des Ru-SILP-Systems ist die Selektivität zum gewünschten Aldehyd 1-Pentanal wesentlich geringer. Hier beträgt die Selektivität zu 1-Pentanal max. 13 %. Die Zugabe von Liganden wie Triphenylphosphine oder Tris(2,4-ditert-butylphenyl)phosphit führt sogar zu einer weiteren Abnahme der Selektivität. Auch die Hydrieraktivität nimmt mit dem kombinierten System SILP-9 und SILP-10 im Vergleich zu den Ruthenium-SILP-Systemen ab. Bei den Systemen SILP-2 und SILP-5, bei denen zusätzlich Ligand eingesetzt wurde, werden zwischen 50-60 % des Butens zu Butan hydriert. In Abwesenheit von Liganden nimmt die Hydrieraktivität stark ab und es werden nur noch 10 % des Butens zu Butan umgesetzt. Durch das kombinierte Katalysatorsystem SILP-9 und SILP-10 kann die Hydrieraktivitäten noch weiter abgesenkt werden, so dass nur noch 5 % Buten hydriert werden.

Der Umsatz aller SILP-Systeme ist auf Grund der Reaktionsbedingungen wie kurze Verweilzeit sehr gering. Dennoch kann im Falle des kombinierten Katalysatorsystems SILP-9 und SILP-10 eine Zunahme des Umsatzes im Vergleich zu SILP-2 und SILP-3 beobachtet werden. Dieser wird um das 2,5-fache gesteigert. Bei SILP-5 ist der Umsatz nahezu identisch, jedoch ist die Selektivität zum gewünschten Aldehyd wesentlich geringer, so dass auch hier mit dem kombinierten Katalysatorsystem SILP-9 und SILP-10 die Gesamtausbeute an Aldehyd höher ist.

Somit kann gezeigt werden, dass das kombinierte Katalysatorsystem aus Rhodium und Ruthenium für die Hydroformylierung von Alkenen mit CO₂ eine bessere Performance als die Ruthenium-SILP-Systeme aufweist und deswegen für die Hydroformylierung mit CO₂ besser geeignet ist.

Tabelle 3 zeigt die Ergebnisse der Charakterisierung der Katalysatorsysteme und des Trägermaterials Silica 60 und Silica 90 mittels BET.

**Tabelle 3: Charakterisierung Trägermaterial**

| | A_{BET} [m²/g] | Porenvolumen [m³/g] | Porendurchmesser [nm] | α |
|---|---|---|---|---|
| *Silica 60* | 492 | 0,72 | 5,9 | - |
| *Silica 90* | 391 | 0,88 | 8,9 | - |
| SILP-2 | 330 | 0,52 | 6,3 | 0,21 |
| SILP-3 | 365 | 0,56 | 6,1 | 0,17 |
| SILP-5 | 304 | 0,46 | 6,0 | 0,2 |
| SILP-9 | 361 | 0,58 | 6,4 | 0,17 |
| SILP-10 | 35 | 0,1 | 11,2 | 0,1* |

| | | | | |
|---|---|---|---|---|
| *: bei SILP-10 bezieht sich das α nur auf die ionische Flüssigkeit. Der voluminöse Stabilisator wird nicht berücksichtigt. | | | | |

### Analytik:

Tabelle 4 zeigt die GC-Parameter zur Analyse der Proben der Hydroformylierung mit dem 450-GC von Bruker.

Tabelle 5 zeigt die GC-Parameter zur Analyse der Proben der Reverse-Wassergas-Shift-Reaktion mit dem 450-GC von Brucker

**Tabelle 4: GC-Parameter Analyse Hydroformylierung**

| | |
|---|---|
| Trägergas | Helium 4.6 (1,3 ml min-1; Split: 1:30) |
| Detektor | Flammenionisationsdetektor (FID) |
| Kapillarsäule | Ultra 2 (50 m x 0,32 mm ID x 0,5 µm DF) |
| Temperaturen | Injektor: 220°C, Detektor: 300°C |
| Temperaturprogramm | isotherm: 5°C für 4 min, aufheizen auf 200°C (10°C min-1) |
| Injektionsvolumen | 250 µl (gasförmig) |
| Auswertungssoftware | Galaxie Chromatography Data System Version 1.9 |

**Tabelle 5: GC-Parameter Analyse Reverse-Wassergas-Shift-Reaktion**

| | |
|---|---|
| Trägergas | Helium 4.6 |
| Detektor | Wärmeleitfähigkeitsdetektor (WLD) |
| Kapillarsäule | ShinCarbon ST 80/100 (2 m x 2 mm ID SILCO) |
| Temperaturen | Injektor: 100 °C, Detektor: 250 °C, Filamenttemperatur: 390 °C |
| Temperaturprogramm | isotherm: 40 °C für 3 min, aufheizen auf 250 °C (8 °C min⁻¹) |
| | isotherm: 250 °C für 30 min |
| Druckprogramm | isobar: 193 kPa (28 psi) für 3 min, aufheizen auf 317,16 kPa (46 psi) (1,3 psi min⁻¹/ 8,963 kPa min-1) |
| | isobar: 317,16 kPa (46 psi) für 30 min |
| Injektionsvolumen | 250 µl (gasförmig) |
| Auswertungssoftware | Galaxie Chromatography Data System Version 1.9 |

## Patentansprüche

1. Verfahren zur Herstellung von Aldehyden aus Alkenen und Kohlendioxid,
bei welchem zunächst Kohlendioxid mit Wasserstoff im Wege einer Reverse-Wassergas-Shift-Reaktion zu Kohlenmonoxid und Wasser umgesetzt wird,
und sodann mindestens ein Alken mit dem erhaltenen Kohlenmonoxid und mit Wasserstoff im Wege einer Hydroformylierung zu mindestens einem korrespondierenden Aldehyd umgesetzt wird,
und bei welchem sowohl die Reverse-Wassergas-Shift-Reaktion, als auch die Hydroformylierung in Gegenwart eines Katalysator-Systems erfolgt, welches als SILP (Supported Ion Liquid Phase)-Katalysator-System eine katalytisch wirksame Zusammensetzung umfasst, die in einer einen festen Träger benetzenden, ionischen Flüssigkeit gelöst ist, **dadurch gekennzeichnet,**
**dass** zwei unterschiedliche SILP-Katalysator-Systeme eingesetzt werden, nämlich ein erstes SILP-Katalysator-System, in dessen Gegenwart die Reverse-Wassergas-Shift-Reaktion durchgeführt wird,
und ein zweites SILP-Katalysator-System, in dessen Gegenwart die Hydroformylierung durchgeführt wird,
wobei das erste SILP-Katalysator-System einen in ionischer Flüssigkeit gelösten Ruthenium-Komplex als katalytisch wirksame Zusammensetzung umfasst,
und wobei das zweite SILP-Katalysator-System einen in ionischer Flüssigkeit gelösten Rhodium-Komplex als katalytisch wirksame Zusammensetzung umfasst.

2. Verfahren nach Anspruch 1, umfassend die folgenden Schritte:
a) Beaufschlagen einer ersten Schüttung enthaltend das erste SILP-Katalysator-System mit einem ersten Eduktgasstrom enthaltend zumindest Kohlendioxid und Wasserstoff;
b) Abziehen eines ersten Produktgasstroms enthaltend zumindest Kohlenmonoxid und Wasser aus der ersten Schüttung;
c) Beaufschlagen einer zweiten Schüttung enthaltend das zweite SILP-Katalysator-System mit einem zweiten Eduktgasstrom enthaltend zumindest Kohlenmonoxid, Wasserstoff und Alken, wobei zumindest ein Teil des enthaltenden Kohlenmonoxids aus dem ersten Produktgasstrom stammt;
d) Abziehen eines zweiten Produktgasstroms enthaltend zumindest ein korrespondierendes Aldehyd aus der zweiten Schüttung.

3. Verfahren nach Anspruch 1, umfassend die folgenden Schritte:
a) Beaufschlagen einer ersten Schüttung enthaltend das erste SILP-Katalysator-System mit einem ersten Eduktgasstrom enthaltend zumindest Kohlendioxid und Wasserstoff;
b) Abziehen eines ersten Produktgasstroms enthaltend zumindest Kohlenmonoxid und Wasser aus der ersten Schüttung;
c) Entfernen zumindest eines Teils des im ersten Produktgasstrom enthaltenden Wassers mit Hilfe eines Adsorbers;
d) Beaufschlagen einer zweiten Schüttung enthaltend das zweite SILP-Katalysator-System mit einem zweiten Eduktgasstrom enthaltend zumindest Kohlenmonoxid, Wasserstoff und Alken, wobei zumindest ein Teil des enthaltenden Kohlenmonoxids aus dem ersten Produktgasstrom stammt;
e) Abziehen eines zweiten Produktgasstroms enthaltend zumindest ein korrespondierendes Aldehyd aus der zweiten Schüttung.

4. Verfahren nach Anspruch 1, umfassend die folgenden Schritte:
a) Beaufschlagen einer ersten Schüttung enthaltend das erste SILP-Katalysator-System mit einem ersten Eduktgasstrom enthaltend zumindest Kohlendioxid und Wasserstoff;
b) Abziehen eines ersten Produktgasstroms enthaltend zumindest Kohlenmonoxid und Wasser aus der ersten Schüttung;
c) Entfernen zumindest eines Teils des im ersten Produktgasstrom enthaltenden Wassers mit Hilfe eines Adsorbers;
d) Zugabe von zumindest Alken und Wasserstoff in den entwässerten ersten Produktgasstrom unter Erhalt eines zweiten Eduktgasstroms;
e) Beaufschlagen einer zweiten Schüttung enthaltend das zweite SILP-Katalysator-System mit dem zweiten Eduktgasstrom enthaltend zumindest Kohlenmonoxid, Wasserstoff und Alken, wobei zumindest ein Teil des enthaltenden Kohlenmonoxids aus dem ersten Produktgasstrom stammt;
f) Abziehen eines zweiten Produktgasstroms enthaltend zumindest ein korrespondierendes Aldehyd aus der zweiten Schüttung.

5. Verfahren nach Anspruch 1, umfassend die folgenden Schritte:
a) Beaufschlagen einer ersten Schüttung enthaltend das erste SILP-Katalysator-System mit einem ersten Eduktgasstrom enthaltend zumindest Kohlendioxid und ein Überschuss an Wasserstoff;
b) Abziehen eines ersten Produktgasstroms enthaltend zumindest unumgesetzten Wasserstoff, Kohlenmonoxid und Wasser aus der ersten Schüttung;
c) Entfernen zumindest eines Teils des im ersten Produktgasstrom enthaltenden Wassers mit Hilfe eines Adsorbers;
d) Zugabe von zumindest Alken in den entwässerten ersten Produktgasstrom unter Erhalt eines zweiten Eduktgasstroms;
e) Beaufschlagen einer zweiten Schüttung enthaltend das zweite SILP-Katalysator-System mit dem zweiten Eduktgasstrom enthaltend zumindest Kohlenmonoxid, Wasserstoff und Alken, wobei zumindest ein Teil des enthaltenden Kohlenmonoxids aus dem ersten Produktgasstrom stammt;
f) Abziehen eines zweiten Produktgasstroms enthaltend zumindest ein korrespondierendes Aldehyd aus der zweiten Schüttung.

6. Verfahren nach Anspruch 1, umfassend die folgenden Schritte:
a) Beaufschlagen einer ersten Schüttung enthaltend das erste SILP-Katalysator-System mit einem ersten Eduktgasstrom enthaltend zumindest Kohlendioxid, Alken und ein Überschuss an Wasserstoff;
b) Abziehen eines ersten Produktgasstroms enthaltend zumindest unumgesetzten Wasserstoff, Alken, Kohlenmonoxid und Wasser aus der ersten Schüttung;
c) Entfernen zumindest eines Teils des im ersten Produktgasstrom enthaltenden Wassers mit Hilfe eines Adsorbers unter Erhalt eines zweiten Eduktgasstroms;
d) Beaufschlagen einer zweiten Schüttung enthaltend das zweite SILP-Katalysator-System mit dem zweiten Eduktgasstrom enthaltend zumindest Kohlenmonoxid, Wasserstoff und Alken, wobei zumindest ein Teil des enthaltenden Kohlenmonoxids aus dem ersten Produktgasstrom stammt;
e) Abziehen eines zweiten Produktgasstroms enthaltend zumindest ein korrespondierendes Aldehyd aus der zweiten Schüttung.

7. Verfahren nach einem der vorhergehenden Ansprüche, **dadurch gekennzeichnet, dass** das erste SILP-Katalysator-System als Ruthenium-Komplex Trirutheniumdodecacarbonyl (Ru₃(CO)₁₂) enthält, welcher gelöst ist in einer ionischen Flüssigkeit ausgewählt aus:
1-Ethyl-3-methylimidazolium bis(triflouoromethansulfonyl)imide ([EMIM][Ntf2]),
1-Butyl-3-methylimidazolium bis(triflouoromethansulfonyl)imide ([BMIM][Ntf2]),
1-Ethyl-3-methylimidazolium chlorid ([EMIM]Cl),
1-Butyl-3-methylimidazolium-chlorid ([BMIM]Cl),
oder Mischungen daraus.

8. Verfahren nach einem der vorhergehenden Ansprüche, **dadurch gekennzeichnet, dass** das zweite SILP-Katalysator-System als Rhodium-Komplex (Acetylacetonato)dicarbonylrhodium (Rh(CO)₂(acac))) enthält, welcher gelöst ist in 1-Ethyl-3-methylimidazolium bis(triflouoromethansulfonyl)imide ([EMIM][Ntf2]), und welcher zusätzlich Triphenylphosphin (PPh3) als Ligand und Bis(2,2,6,6 tetramethyl 4 piperidyl) sebacate (C₁₀H₁₆O₄ (C₉H₁₈N)₂) als Stabilisator enthält.

9. Verfahren nach einem die vorhergehenden Ansprüche, **dadurch gekennzeichnet, dass** Kieselsäure bei mindestens einem der beiden SILP-Katalysator-Systeme als Träger verwendet wird.

10. Verfahren nach einem die vorhergehenden Ansprüche, **dadurch gekennzeichnet, dass** mindestens ein Alken mit vier Kohlenstoffatomen eingesetzt wird, welches zu dem korrespondierenden Aldehyd mit fünf Kohlenstoffatomen hydroformyliert wird.

## Claims

1. Process for preparing aldehydes from alkenes and carbon dioxide, in which carbon dioxide is firstly reacted with hydrogen in the manner of a reverse water gas shift reaction to form carbon monoxide and water and subsequently at least one alkene is reacted with the carbon monoxide obtained and hydrogen in the manner of a hydroformylation to form at least one corresponding aldehyde,
and in which both the reverse water gas shift reaction and the hydroformylation are carried out in the presence of a catalyst system which comprises a catalytic reactive composition dissolved in an ionic liquid which wets a solid support as SILP (supported ion liquid phase) catalyst system, **characterized**
**in that** two different SILP catalyst systems are used, namely a first SILP catalyst system in whose presence the reverse water gas shift reaction is carried out
and a second SILP catalyst system in whose presence the hydroformylation is carried out,
where the first SILP catalyst system comprises a ruthenium complex dissolved in an ionic liquid as catalytically active composition and the second SILP catalyst system comprises a rhodium complex dissolved in an ionic liquid as catalytically active composition.

2. Process according to Claim 1, which comprises the following steps:
a) supplying a first bed containing the first SILP catalyst system with a first feed gas stream containing at least carbon dioxide and hydrogen;
b) drawing off a first product gas stream containing at least carbon monoxide and water from the first bed;
c) supplying a second bed containing the second SILP catalyst system with a second feed gas stream containing at least carbon monoxide, hydrogen and alkene, with at least part of the carbon monoxide present originating from the first product gas stream;
d) drawing off a second product gas stream containing at least one corresponding aldehyde from the second bed.

3. Process according to Claim 1, which comprises the following steps:
a) supplying a first bed containing the first SILP catalyst system with a first feed gas stream containing at least carbon dioxide and hydrogen;
b) drawing off a first product gas stream containing at least carbon monoxide and water from the first bed;
c) removing at least part of the water present in the first product gas stream by means of an absorber;
d) supplying a second bed containing the second SILP catalyst system with a second feed gas stream containing at least carbon monoxide, hydrogen and alkene, with at least part of the carbon monoxide present originating from the first product gas stream;
e) drawing off a second product gas stream containing at least one corresponding aldehyde from the second bed.

4. Process according to Claim 1, which comprises the following steps:
a) supplying a first bed containing the first SILP catalyst system with a first feed gas stream containing at least carbon dioxide and hydrogen;
b) drawing off a first product gas stream containing at least carbon monoxide and water from the first bed;
c) removing at least part of the water present in the first product gas stream by means of an adsorber;
d) introducing at least alkene and hydrogen into the dewatered first product gas stream to give a second feed gas stream;
e) supplying a second bed containing the second SILP catalyst system with the second feed gas stream containing at least carbon monoxide, hydrogen and alkene, with at least part of the carbon monoxide present originating from the first product gas stream;
f) drawing off a second product gas stream containing at least one corresponding aldehyde from the second bed.

5. Process according to Claim 1, which comprises the following steps:
a) supplying a first bed containing the first SILP catalyst system with a first feed gas stream containing at least carbon dioxide and an excess of hydrogen;
b) drawing off a first product gas stream containing at least unreacted hydrogen, carbon monoxide and water from the first bed;
c) removing at least part of the water present in the first product gas stream by means of an adsorber;
d) introducing at least alkene into the dewatered first product gas stream to give a second feed gas stream;
e) supplying a second bed containing the second SILP catalyst system with the second feed gas stream containing at least carbon monoxide, hydrogen and alkene, with at least part of the carbon monoxide present originating from the first product gas stream;
f) drawing off a second product gas stream containing at least one corresponding aldehyde from the second bed.

6. Process according to Claim 1, which comprises the following steps:
a) supplying a first bed containing the first SILP catalyst system with a first feed gas stream containing at least carbon dioxide, alkene and an excess of hydrogen;
b) drawing off a first product gas stream containing at least unreacted hydrogen, alkene, carbon monoxide and water from the first bed;
c) removing at least part of the water present in the first product gas stream by means of an adsorber to give a second feed gas stream;
d) supplying a second bed containing the second SILP catalyst system with the second feed gas stream containing at least carbon monoxide, hydrogen and alkene, with at least part of the carbon monoxide present originating from the first product gas stream;
e) drawing off a second product gas stream containing at least one corresponding aldehyde from the second bed.

7. Process according to any of the preceding claims, **characterized in that** the first SILP catalyst system contains triruthenium dodecacarbonyl (Ru₃(CO)₁₂) as ruthenium complex dissolved in an ionic liquid selected from among:
1-ethyl-3-methylimidazolium bis(trifluoromethanesulfonyl)imide ([EMIM][Ntf2]),
1-butyl-3-methylimidazolium bis(trifluoromethanesulfonyl)imide ([BMIM][Ntf2]),
1-ethyl-3-methylimidazolium chloride ([EMIM]Cl),
1-butyl-3-methylimidazolium chloride ([BMIM]Cl)
and mixtures thereof.

8. Process according to any of the preceding claims, **characterized in that** the second SILP catalyst system contains (acetylacetonato)dicarbonylrhodium (Rh(CO)₂(acac))) as rhodium complex dissolved in 1-ethyl-3-methylimidazolium bis(trifluoromethanesulfonyl)imide ([EMIM][Ntf2]) and additionally contains triphenylphosphine (PPh3) as a ligand and bis(2,2,6,6-tetramethyl-4-piperidyl) sebacate (C₁₀H₁₆O₄ (C₉H₁₈N)₂) as stabiliser.

9. Process according to any of the preceding claims, **characterized in that** silica is used as support in at least one of the two SILP catalyst systems.

10. Process according to any of the preceding claims, **characterized in that** at least one alkene which has four carbon atoms and is hydroformylated to the corresponding aldehyde having five carbon atoms is used.

## Revendications

1. Procédé de fabrication d'aldéhydes à partir d'alcènes et de dioxyde de carbone, selon lequel du dioxyde de carbone est tout d'abord mis en réaction avec de l'hydrogène par une réaction du gaz à l'eau inverse pour former du monoxyde de carbone et de l'eau, puis au moins un alcène est mis en réaction avec le monoxyde de carbone obtenu et avec de l'hydrogène par une hydroformylation pour former au moins un aldéhyde correspondant,
et selon lequel aussi bien la réaction du gaz à l'eau inverse que l'hydroformylation ont lieu en présence d'un système catalytique qui comprend en tant que système catalytique SILP (Supported Ion Liquid Phase) une composition catalytiquement active, qui est dissoute dans un liquide ionique mouillant un support solide, **caractérisé en ce que** deux systèmes catalytiques SILP différents sont utilisés, à savoir un premier système catalytique SILP en présence duquel la réaction du gaz à l'eau inverse est réalisée, et un second système catalytique SILP en présence duquel l'hydroformylation est réalisée,
le premier système catalytique SILP comprenant un complexe de ruthénium dissous dans un liquide ionique en tant que composition catalytiquement active, et le second système catalytique SILP comprenant un complexe de rhodium dissous dans un liquide ionique en tant que composition catalytiquement active.

2. Procédé selon la revendication 1, comprenant les étapes suivantes :
a) le chargement d'un premier garnissage contenant le premier système catalytique SILP avec un premier courant gazeux de réactifs contenant au moins du dioxyde de carbone et de l'hydrogène ;
b) le soutirage d'un premier courant gazeux de produits contenant au moins du monoxyde de carbone et de l'eau du premier garnissage ;
c) le chargement d'un second garnissage contenant le second système catalytique SILP avec un second courant gazeux de réactifs contenant au moins du monoxyde de carbone, de l'hydrogène et un alcène, au moins une partie du monoxyde de carbone contenu provenant du premier courant gazeux de produits ;
d) le soutirage d'un second courant gazeux de produits contenant au moins un aldéhyde correspondant du second garnissage.

3. Procédé selon la revendication 1, comprenant les étapes suivantes :
a) le chargement d'un premier garnissage contenant le premier système catalytique SILP avec un premier courant gazeux de réactifs contenant au moins du dioxyde de carbone et de l'hydrogène ;
b) le soutirage d'un premier courant gazeux de produits contenant au moins du monoxyde de carbone et de l'eau du premier garnissage ;
c) l'élimination d'au moins une partie de l'eau contenue dans le premier courant gazeux de produits à l'aide d'un adsorbeur ;
d) le chargement d'un second garnissage contenant le second système catalytique SILP avec un second courant gazeux de réactifs contenant au moins du monoxyde de carbone, de l'hydrogène et un alcène, au moins une partie du monoxyde de carbone contenu provenant du premier courant gazeux de produits ;
e) le soutirage d'un second courant gazeux de produits contenant au moins un aldéhyde correspondant du second garnissage.

4. Procédé selon la revendication 1, comprenant les étapes suivantes :
a) le chargement d'un premier garnissage contenant le premier système catalytique SILP avec un premier courant gazeux de réactifs contenant au moins du dioxyde de carbone et de l'hydrogène ;
b) le soutirage d'un premier courant gazeux de produits contenant au moins du monoxyde de carbone et de l'eau du premier garnissage ;
c) l'élimination d'au moins une partie de l'eau contenue dans le premier courant gazeux de produits à l'aide d'un adsorbeur ;
d) l'ajout d'au moins un alcène et d'hydrogène au premier courant gazeux de produits déshydraté pour obtenir un second courant gazeux de réactifs ;
e) le chargement d'un second garnissage contenant le second système catalytique SILP avec le second courant gazeux de réactifs contenant au moins du monoxyde de carbone, de l'hydrogène et un alcène, au moins une partie du monoxyde de carbone contenu provenant du premier courant gazeux de produits ;
f) le soutirage d'un second courant gazeux de produits contenant au moins un aldéhyde correspondant du second garnissage.

5. Procédé selon la revendication 1, comprenant les étapes suivantes :
a) le chargement d'un premier garnissage contenant le premier système catalytique SILP avec un premier courant gazeux de réactifs contenant au moins du dioxyde de carbone et un excès d'hydrogène ;
b) le soutirage d'un premier courant gazeux de produits contenant au moins de l'hydrogène non réagi, du monoxyde de carbone et de l'eau du premier garnissage ;
c) l'élimination d'au moins une partie de l'eau contenue dans le premier courant gazeux de produits à l'aide d'un adsorbeur ;
d) l'ajout d'au moins un alcène au premier courant gazeux de produits déshydraté pour obtenir un second courant gazeux de réactifs ;
e) le chargement d'un second garnissage contenant le second système catalytique SILP avec le second courant gazeux de réactifs contenant au moins du monoxyde de carbone, de l'hydrogène et un alcène, au moins une partie du monoxyde de carbone contenu provenant du premier courant gazeux de produits ;
f) le soutirage d'un second courant gazeux de produits contenant au moins un aldéhyde correspondant du second garnissage.

6. Procédé selon la revendication 1, comprenant les étapes suivantes :
a) le chargement d'un premier garnissage contenant le premier système catalytique SILP avec un premier courant gazeux de réactifs contenant au moins du dioxyde de carbone, un alcène et un excès d'hydrogène ;
b) le soutirage d'un premier courant gazeux de produits contenant au moins de l'hydrogène non réagi, un alcène, du monoxyde de carbone et de l'eau du premier garnissage ;
c) l'élimination d'au moins une partie de l'eau contenue dans le premier courant gazeux de produits à l'aide d'un adsorbeur pour obtenir un second courant gazeux de réactifs ;
d) le chargement d'un second garnissage contenant le second système catalytique SILP avec le second courant gazeux de réactifs contenant au moins du monoxyde de carbone, de l'hydrogène et un alcène, au moins une partie du monoxyde de carbone contenu provenant du premier courant gazeux de produits ;
e) le soutirage d'un second courant gazeux de produits contenant au moins un aldéhyde correspondant du second garnissage.

7. Procédé selon l'une quelconque des revendications précédentes, **caractérisé en ce que** le premier système catalytique SILP contient en tant que complexe de ruthénium du triruthéniumdodécacarbonyle (Ru₃(CO)₁₂), qui est dissous dans un liquide ionique choisi parmi :
les bis(trifluorométhanesulfonyl)imides de 1-éthyl-3-méthylimidazolium ([EMIM][Ntf2]),
les bis(trifluorométhanesulfonyl)imides de 1-butyl-3-méthylimidazolium ([BMIM][Ntf2]),
le chlorure de 1-éthyl-3-méthylimidazolium ([EMIM]Cl),
le chlorure de 1-butyl-3-méthylimidazolium ([BMIM]Cl),
ou leurs mélanges.

8. Procédé selon l'une quelconque des revendications précédentes, **caractérisé en ce que** le second système catalytique SILP contient en tant que complexe de rhodium de l'(acétylacétonato)dicarbonylrhodium (Rh(CO)₂)(acac)), qui est dissous dans des bis(trifluorométhanesulfonyl)imides de 1-éthyl-3-méthylimidazolium ([EMIM][Ntf2]), et qui contient en outre de la triphénylphosphine (PPh3) en tant que ligand et des sébacates de bis(2,2,6,6-tétraméthyl-4-pipéridyle) (C₁₀H₁₆O₄(C₉H₁₈N)₂) en tant que stabilisateur.

9. Procédé selon l'une quelconque des revendications précédentes, **caractérisé en ce que** de la silice est utilisée dans au moins un des deux systèmes catalytiques SILP en tant que support.

10. Procédé selon l'une quelconque des revendications précédentes, **caractérisé en ce qu'**au moins un alcène contenant quatre atomes de carbone est utilisé, qui est hydroformylé en l'aldéhyde correspondant contenant cinq atomes de carbone.
